(19)

**Europäisches Patentamt**

**European Patent Office**

**Office européen des brevets**

(11) **EP 4 574 965 A1**

(12) **EUROPEAN PATENT APPLICATION**

(43) Date of publication:
25.06.2025 Bulletin 2025/26

(21) Application number: 24214217.2

(22) Date of filing: 20.11.2024

(51) International Patent Classification (IPC):
*C12N 1/20* $^{(2006.01)}$

(52) Cooperative Patent Classification (CPC):
**C12N 1/20; C12N 1/205;** C12R 2001/07

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: 22.12.2023 TW 112150243

(71) Applicant: Sinon Corporation
Taichung City 432402 (TW)

(72) Inventors:
• TSAI, JERRY
432402 TAICHUNG CITY (TW)
• HU, CHIH HSIEN
432402 TAICHUNG CITY (TW)
• LIN, CHIA HSIEN
432402 TAICHUNG CITY (TW)

(74) Representative: Straus, Alexander
2K Patentanwälte - München
Bajuwarenring 14
82041 Oberhaching (DE)

(54) **METHOD FOR LARGE-SCALE PRODUCTION OF FERMENTATION BROTH WITH HIGH CONCENTRATION OF BACILLUS VELEZENSIS STRAIN, FERMENTATION BROTH PRODUCED BY THE METHOD, AND LIQUID MEDIUM FOR THE METHOD**

(57) A method for large-scale production of a fermentation broth with a high concentration of *Bacillus velezensis* strain includes the steps of inoculating a seed broth of *Bacillus velezensis* strain with a bacterial count of $1\times10^7$ to $1\times10^9$ CFU/mL at 1% to 10% inoculation ratio into a liquid medium, and culturing in a ton-scale fermentation tank at a temperature of 30°C to 35°C and at a stirring rate of 40 to 70 rpm for 4 to 5 days, to obtain the fermentation broth having a bacterial count of more than $1\times10^9$ CFU/mL. Also provided is a fermentation broth produced by the foregoing production method and a liquid medium for use in the foregoing production method.

**EP 4 574 965 A1**

## Description

### REFERENCE TO A DEPOSIT OF BIOLOGICAL MATERIAL

This application contains a reference to a deposit of biological material, which deposit is incorporated herein by reference.

### TECHNICAL FIELD

The present invention relates to a production method of bacillus fermentation broth and more particularly, to a method for large-scale production of a fermentation broth with a high concentration of *Bacillus velezensis* strain.

### BACKGROUND ART

With the public's attention to food safety and environmental awareness, it has become a global trend to reduce the use of chemical pesticides, and instead use microbial pesticides made from processing of microorganisms (including bacteria, fungi, protozoa, and viruses) and their metabolites, which not only provide the effects of insecticidal, bactericidal, weeding, etc., but also help regulate plant growth.

Since *Bacillus velezensis* strain exhibits antimicrobial activity against phytopathogens such as *Rhizoctonia solani, Nalanthamala psidii, Phytophthora capsici, Pyricularia Oryzae, Corynespora cassiicola, Colletotrichum gloeosporioides, Sclerotium rolfsii,* etc., it can effectively inhibit pathogen growth. Therefore, it is suitable for use as a microbial pesticide.

Currently, a method for enrichment culture of *Bacillus velezensis* KHH13 strain exists, as described in Taiwan Patent No. I740263. However, small-capacity fermentation tanks in laboratories cannot meet the industrial mass production needs. The primary challenge in large-scale microbial production, especially when using ton-scale fermentation equipment, is how to stably and effectively increase the bacterial count of microorganisms.

### SUMMARY OF THE INVENTION

In view of this, an objective of the present invention is to provide a method for producing a fermentation broth of *Bacillus velezensis* strain, which is not only suitable for ton-scale fermentation equipment but also capable of producing a fermentation broth having a bacterial count of more than $1 \times 10^9$ CFU/mL in a cost-effective manner. Additionally, the obtained fermentation broth exhibits excellent storage stability.

Another objective of the present invention is to provide a *Bacillus velezensis* strain fermentation broth produced by the aforementioned production method.

A further objective of the present invention is to provide a liquid medium suitable for use in the aforementioned production method.

To achieve the objectives disclosed above, one aspect of the present invention provides a method for large-scale production of a fermentation broth with a high concentration of *Bacillus velezensis* strain. The method comprises the steps of inoculating a seed broth of *Bacillus velezensis* strain having a bacterial count of $1 \times 10^7$ to $1 \times 10^9$ CFU/mL at 1% to 10% inoculation ratio into a liquid medium, and culturing in a ton-scale fermentation tank at a temperature of 30°C to 35°C and at a stirring rate of 40 to 70 rpm for 4 to 5 days, to obtain the fermentation broth having a bacterial count of more than $1 \times 10^9$ CFU/mL. The *Bacillus velezensis* strain used in the method is deposited at the China Center for Type Culture Collection (CCTCC) under accession number M20211597 and is also deposited at the Bioresource Collection and Research Center (BCRC) of Food Industry Research and Development Institute (FIRDI) in Taiwan under accession number BCRC MP10008. The liquid medium comprises 0.5 to 1 wt% of carbon source, 1 to 2.5 wt% of nitrogen source, 0.1 to 0.3 wt% of nitrate compound, 0.01 to 0.05 wt% of chlorine source, 0.005 to 0.010 wt% of calcium source, 0.05 to 0.15 wt% of magnesium source and a balance of deionized water.

According to one embodiment of the present invention, the obtained fermentation broth has a bacterial count of less than $3 \times 10^{10}$ CFU/mL.

According to one embodiment of the present invention, in the liquid medium, the carbon source is any one or a mixture of at least two selected from glucose, molasses, granulated sugar, lactose, or corn starch; and the nitrogen source is any one or a mixture of at least two selected from yeast powder, full-fat soybean powder, defatted soybean powder, soybean protein, or peptone.

According to another embodiment of the present invention, in the liquid medium, the nitrate compound is ammonium nitrate, potassium nitrate, or a mixture thereof; the chlorine source is any one or a mixture of at least two selected from sodium chloride, ferric chloride, or potassium chloride; the calcium source is calcium carbonate, monocalcium phosphate, or a mixture thereof; and the magnesium source is magnesium chloride, magnesium sulfate, or a mixture thereof.

According to a further embodiment of the present invention, when the bacterial count in the seed broth is $1 \times 10^8$ to $1 \times 10^9$ CFU/mL and the liquid medium comprises 1 wt% of glucose, 2.5 wt% of yeast powder, 0.05 wt% of potassium chloride, 0.3 wt% of potassium nitrate, 0.010 wt% of calcium carbonate, 0.07 wt% of magnesium chloride, and a balance of deionized water, the bacterial count in the obtained fermentation broth is in a range of $1 \times 10^{10}$ CFU/mL to $3 \times 10^{10}$ CFU/mL.

To achieve the objectives disclosed above, another aspect of the present invention provides a *Bacillus velezensis* strain fermentation broth having a bacterial count ranging from $1 \times 10^9$ CFU/mL to $3 \times 10^{10}$ CFU/mL, which is produced by the production method.

To achieve the objectives disclosed above, yet another aspect of the present invention provides a liquid medium for use in the production method, enabling large-scale production of a fermentation broth with a high concentration of *Bacillus velezensis* strain. The liquid medium comprises 0.5 to 1 wt% of carbon source, 1 to 2.5 wt% of nitrogen source, 0.1 to 0.3 wt% of nitrate compound, 0.01 to 0.05 wt% of chlorine source, 0.005 to 0.010 wt% of calcium source, 0.05 to 0.15 wt% of magnesium source, and a balance of deionized water.

According to one embodiment of the present invention, in the liquid medium, the carbon source is any one or a mixture of at least two selected from glucose, molasses, granulated sugar, lactose, or corn starch; the nitrogen source is any one or a mixture of at least two selected from yeast powder, full-fat soybean powder, defatted soybean powder, soybean protein, or peptone.

According to another embodiment of the present invention, in the liquid medium, the nitrate compound is ammonium nitrate, potassium nitrate, or a mixture thereof; the chlorine source is any one or a mixture of at least two selected from sodium chloride, ferric chloride, or potassium chloride; the calcium source is calcium carbonate, monocalcium phosphate, or a mixture thereof; and the magnesium source is magnesium chloride, magnesium sulfate, or a mixture thereof.

According to still another embodiment of the present invention, in the liquid medium, the carbon source is 0.5 to 1 wt% of glucose, the nitrogen source is 1 to 2.5 wt% of yeast powder, the nitrate compound is 0.3 wt% of potassium nitrate, the chlorine source is 0.05 wt% of potassium chloride, the calcium source is 0.01 wt% of calcium carbonate, and the magnesium source is 0.07 wt% of magnesium chloride.

Accordingly, the production method of the present invention can utilize ton-scale fermentation equipment to large scale (tons) produce a fermentation broth under cost-effective conditions, and the obtained fermentation broth contains a bacterial count of more than $1 \times 10^9$ CFU/mL, even up to $10^{10}$ CFU/mL. Therefore, the production method not only meets the industrial mass production needs, but also can efficiently produce a fermentation broth having high bacterial count. Additionally, since the obtained fermentation broth exhibits excellent storage stability, it is quite beneficial for subsequent applications of microbial pesticides.

## DETAILED DESCRIPTION OF EMBODIMENTS

The following will describe the technology of the present invention in more detail in combination with specific embodiments of the present invention. However, the described embodiments and/or examples are only parts of embodiments and/or examples of the present invention and not all the embodiments and/or examples. Various modifications and variations made by persons of ordinary skill in the art based on embodiments and/or examples disclosed in the present invention without departing from the spirit of the present invention are within the scope of the present invention.

The present invention provides a production method of a fermentation broth of *Bacillus velezensis* strain, which comprises the following steps:

inoculating a seed broth of *Bacillus velezensis* strain having a bacterial count of $1 \times 10^7$ to $1 \times 10^9$ CFU/mL at 1% to 10% inoculation ratio into a liquid medium, and culturing in a ton-scale fermentation tank at a temperature of 30°C to 35°C and at a stirring rate of 40 to 70 rpm for 4 to 5 days, to obtain the fermentation broth having a bacterial count of more than $1 \times 10^9$ CFU/mL;

wherein the *Bacillus velezensis* strain is deposited at the China Center for Type Culture Collection (CCTCC) under accession number M20211597 and is also deposited at the Bioresource Collection and Research Center (BCRC) of Food Industry Research and Development Institute (FIRDI) in Taiwan under accession number BCRC MP10008; and

the liquid medium comprises 0.5 to 1 wt% of carbon source, 1 to 2.5 wt% of nitrogen source, 0.1 to 0.3 wt% of nitrate compound, 0.01 to 0.05 wt% of chlorine source, 0.005 to 0.010 wt% of calcium source, 0.05 to 0.15 wt% of magnesium source, and a balance of deionized water.

**Seed Broth**

In the embodiments of the present invention, the seed broth of *Bacillus velezensis* strain having a bacterial count of $1 \times 10^7$ to $1 \times 10^9$ CFU/mL can be obtained by conventional methods, without specific limitation. For example, the seed broth can be obtained via the following method:

take out the *Bacillus velezensis* strain (CCTCC, accession number M20211597) seed source from a -80°C strain library and inoculate the seed source into an LB (Luria-Bertani Broth) liquid medium. The culture is carried out at a temperature of 25 to 35°C and at a stirring rate of 100 to 200 rpm for 17 to 24 hours, to obtain a primary seed suspension. Then, inoculate the primary seed suspension into a liquid medium at 1% to 10% inoculation ratio. The liquid fermentation culture is performed in a fermentation tank at a temperature of 25 to 35°C and at a stirring rate of 150 to 350 rpm for 17 to 24 hours, thereby obtaining a seed broth of *Bacillus velezensis* strain having a bacterial count of $1 \times 10^7$ to $1 \times 10^9$ CFU/mL.

The liquid medium used in obtaining the seed broth of *Bacillus velezensis* strain may comprise 0.5 to 1 wt% of carbon source, 1 to 2.5 wt% of nitrogen source, 0.1 to 0.3 wt% of nitrate compound, 0.01 to 0.05 wt% of chlorine source, 0.005 to 0.010 wt% of calcium source, 0.05 to 0.15 wt% of magnesium source, and a balance of deionized water.

**Liquid Medium**

In the embodiments of the present invention, the carbon source contained in each of the liquid media may include, but not limited to, any one or a mixture of at least two of glucose, molasses, granulated sugar, lactose, and corn starch. The nitrogen source contained in each of the liquid media may include, but not limited to, any one or a mixture of at least two of yeast powder, full-fat soybean powder, defatted soybean powder, soybean protein, or peptone. The nitrate compound contained in each of the liquid media may be ammonium nitrate, potassium nitrate, or a mixture thereof. The chlorine source contained in each of the liquid media may include, but not limited to, any one or a mixture of at least two of sodium chloride, ferric chloride, or potassium chloride. The calcium source contained in each of the liquid media may be calcium carbonate, monocalcium phosphate, or a mixture thereof. The magnesium source contained in each of the liquid media may be magnesium chloride, magnesium sulfate, or a mixture thereof.

Preferably, the content of the carbon source in each of the liquid media is 0.5 to 1 wt% and the content of the nitrogen source is 1 to 2.5 wt%. If the content of the carbon source is lower than 0.5 wt% and the content of the nitrogen source is lower than 1 wt%, a fermentation broth having a bacterial count of more than $1 \times 10^9$ CFU/mL cannot be produced. If the content of the carbon source is higher than 1 wt% and the content of the nitrogen source is higher than 2.5 wt%, the bacterial count in the obtained fermentation broth will not increase significantly, but the production costs will rise, thereby reducing the economic efficiency of the present invention.

In the embodiments of the present invention, the inoculation ratio of the seed broth is preferably between 1% to 10%. If the inoculation ratio is lower than 1%, a fermentation broth having a bacterial count of more than $1 \times 10^9$ CFU/mL cannot be produced. If the inoculation ratio is higher than 10%, the bacterial count in the obtained fermentation broth will not increase significantly, thus reducing the economic efficiency of the present invention.

In the embodiments of the present invention, the culturing temperature is preferably between 30°C to 35°C. If the temperature is lower than 30°C, a fermentation broth having a bacterial count of more than $1 \times 10^9$ CFU/mL cannot not be produced. If the temperature is higher than 35°C, the bacterial count in the obtained fermentation broth will not increase significantly, but the energy consumption will rise, thereby reducing the economic efficiency of the present invention.

In the embodiments of the present invention, the stirring rate during the culture is preferably between 40 rpm to 70 rpm. If the stirring rate is lower than 40 rpm, a fermentation broth having a bacterial count of more than $1 \times 10^9$ CFU/mL cannot be produced. If the stirring rate is higher than 70 rpm, the bacterial count in the obtained fermentation broth will not increase significantly, but the energy consumption will rise, thereby reducing the economic efficiency of the present invention.

The technical contents of the present invention are further illustrated below through specific experimental examples. However, these experimental examples are intended only to exemplify the present invention and should not be construed as limiting the present invention.

**(1) Small-Scale Fermentation Tank Experiment**

Due to the higher production costs of ton-scale fermentation, the appropriate composition ratio of the liquid medium used to perform the subsequent ton-scale fermentations is determined first by the experiments conducted in a small-scale fermentation tank, taking into account of economic efficiency.

Preparation of Fermentation Broth

50 mL of seed broth of *Bacillus velezensis* strain (CCTCC, accession number M20211597) having a bacterial

count of $1 \times 10^7$ to $1 \times 10^8$ CFU/mL is added to 5 L of liquid medium (i.e., inoculated with seed broth at 1%). Fermentation is carried out at a temperature of 30°C and at a stirring rate of 150 rpm for 5 days to obtain a fermentation broth, after which the bacterial count of the fermentation broth is measured. In the liquid media, according to the composition ratios shown in Table 1, glucose is used as the carbon source, yeast powder as the nitrogen source, potassium nitrate as the nitrate compound, potassium chloride as the chlorine source, calcium carbonate as the calcium source, and magnesium chloride as the magnesium source. The effects of different contents of carbon source, nitrogen source, nitrate compound, chlorine source, calcium source, and magnesium source on the growth of the strain are tested. Each example is repeated three times. The bacterial count of each fermentation broth is calculated, and the average of the bacterial count of the fermentation broth produced in each example is shown in Table 1.

The glucose, yeast powder, potassium nitrate, potassium chloride, calcium carbonate, and magnesium chloride used in the experiments are respectively purchased from Streber-tech Co., LTD., ENERAD Co., Ltd., Streber-tech Co., LTD., Germany K+S Group, Formosa Plastics Corporation, and Ever Sing Co., Ltd.

Table 1

| Example | liquid medium composition ratio | | | | | | condition | | | FBC |
|---|---|---|---|---|---|---|---|---|---|---|
| | A (wt%) | B (wt%) | C (wt%) | D (wt%) | E (wt%) | F (wt%) | T (°C) | SR (rpm) | IR (%) | BC (cfu/mL) |
| 1 | 0.25 | 0.50 | 0.30 | 0.05 | 0.010 | 0.07 | 30 | 150 | 1.0 | $1.73 \times 10^8$ |
| 2 | 0.25 | 1.00 | 0.30 | 0.05 | 0.010 | 0.07 | 30 | 150 | 1.0 | $2.23 \times 10^8$ |
| 3 | 0.25 | 2.50 | 0.30 | 0.05 | 0.010 | 0.07 | 30 | 150 | 1.0 | $4.07 \times 10^8$ |
| 4 | 0.25 | 3.00 | 0.30 | 0.05 | 0.010 | 0.07 | 30 | 150 | 1.0 | $4.50 \times 10^8$ |
| 5 | 0.50 | 0.50 | 0.30 | 0.05 | 0.010 | 0.07 | 30 | 150 | 1.0 | $1.70 \times 10^8$ |
| 6 | 0.50 | 1.00 | 0.30 | 0.05 | 0.010 | 0.07 | 30 | 150 | 1.0 | $2.50 \times 10^8$ |
| 7 | 0.50 | 1.00 | 0.30 | 0.05 | 0.010 | 0.15 | 30 | 150 | 1.0 | $2.77 \times 10^8$ |
| 8 | 0.50 | 1.00 | 0.10 | 0.01 | 0.005 | 0.05 | 30 | 150 | 1.0 | $2.07 \times 10^8$ |
| 9 | 0.50 | 2.50 | 0.30 | 0.05 | 0.010 | 0.07 | 30 | 150 | 1.0 | $7.70 \times 10^8$ |
| 10 | 0.50 | 3.00 | 0.30 | 0.05 | 0.010 | 0.07 | 30 | 150 | 1.0 | $8.10 \times 10^8$ |
| 11 | 1.00 | 0.50 | 0.30 | 0.05 | 0.010 | 0.07 | 30 | 150 | 1.0 | $2.30 \times 10^8$ |
| 12 | 1.00 | 1.00 | 0.30 | 0.05 | 0.010 | 0.07 | 30 | 150 | 1.0 | $4.73 \times 10^9$ |
| 13 | 1.00 | 2.50 | 0.30 | 0.05 | 0.010 | 0.07 | 30 | 150 | 1.0 | $2.20 \times 10^9$ |
| 14 | 1.00 | 2.50 | 0.30 | 0.05 | 0.010 | 0.15 | 30 | 150 | 1.0 | $2.47 \times 10^9$ |
| 15 | 1.00 | 2.50 | 0.10 | 0.01 | 0.005 | 0.05 | 30 | 150 | 1.0 | $2.20 \times 10^9$ |
| 16 | 1.00 | 3.00 | 0.30 | 0.05 | 0.010 | 0.07 | 30 | 150 | 1.0 | $2.60 \times 10^9$ |
| 17 | 1.50 | 0.50 | 0.30 | 0.05 | 0.010 | 0.07 | 30 | 150 | 1.0 | $3.37 \times 10^8$ |
| 18 | 1.50 | 1.00 | 0.30 | 0.05 | 0.010 | 0.07 | 30 | 150 | 1.0 | $6.43 \times 10^8$ |
| 19 | 1.50 | 2.50 | 0.30 | 0.05 | 0.010 | 0.07 | 30 | 150 | 1.0 | $2.63 \times 10^9$ |
| 20 | 1.50 | 3.00 | 0.30 | 0.05 | 0.010 | 0.07 | 30 | 150 | 1.0 | $3.07 \times 10^9$ |

In Table 1, the A, B, C, D, E, F, T, SR, IR, BC, and FBC represent glucose, yeast powder, potassium nitrate, potassium chloride, calcium carbonate, magnesium chloride, temperature, stirring rate, inoculation ratio, bacterial count, and fermentation broth concentration, respectively.

Calculation of Bacterial Count of Fermentation Broth

40 g of medium powder (tryptone 10 g, yeast extract 5.0 g, sodium chloride 10 g, agar 15 g are included) is completely dissolved in 1,000 mL of distilled water. The resulting solution is then sterilized in an autoclave at 121°C for 25 minutes. After that, the solution is removed and cooled to 50 to 60°C. Then, in a sterile operation platform, the solution is slowly poured into 9-cm petri dishes, with 15 to 20 mL in each dish, to obtain LA media for later use.

Under sterile environment, 100 $\mu$L of the fermentation broth is added to a microcentrifuge tube filled with 900 $\mu$L of sterile water, and oscillated uniformly with a test-tube oscillator to obtain a 10-fold dilution. Using the same method, $10^3$-fold to $10^7$-fold dilutions are obtained, i.e., the dilutions with concentrations ranging from $10^{-3}$ fold to $10^{-7}$ fold.

Dilutions with concentrations ranging from $10^{-3}$ to $10^{-7}$ fold are separately placed, each in a volume of 100 $\mu$L, at the center of the surface of each LA medium. The dilution on the surface of the LA medium is spread evenly using a sterile glass bead. After the surface of the LA medium is dry and the dilution has not flowed, the petri dish was placed in an incubator at 30°C for incubation. After incubating for 24 hours, the bacterial count in each dilution is calculated. Since each example is repeated three times, an average bacterial count is obtained by summing each calculated bacterial count and divided by 3. The original bacterial count is calculated from the average bacterial count according to the following calculation formula. This standard calculates the bacterial count using the ten-fold serial dilution and plate count method.

$$\text{original bacterial count} = \frac{N \times X}{V}$$

N: average bacterial count (cfu/petri dish)

X: dilution factor

V: dilution amount (mL/petri dish)

It can be seen from the results shown in Table 1, Examples 12 to 16, with the bacterial counts up to $10^9$ CFU/mL, are optimal. Examples 9 to 10 followed, with the bacterial counts of $7.70 \times 10^8$ CFU/mL to $8.10 \times 10^8$ CFU/mL. The fermentation broths obtained in Examples 1 and 5 have the lowest bacterial counts of only about $1.70 \times 10^8$ CFU/mL. It is apparent that when the content of yeast powder is 0.5 wt%, the fermentation broth having a higher bacterial count cannot be obtained. In Examples 2, 3, and 6 to 9, when the content of yeast powder ranges from 1 to 2.5 wt% and the content of glucose is 0.5 wt%, the obtained fermentation broths have higher bacterial counts. In Examples 6 to 8, when the content of glucose is 0.5 wt% and the content of yeast powder is 1 wt%, varying the contents of potassium nitrate, potassium chloride, calcium carbonate, and magnesium chloride resulted in little difference in the bacterial counts of the obtained fermentation broths. In addition, although the fermentation broths in Examples 19 and 20 also achieve a bacterial count of $10^9$ CFU/mL, the amounts of glucose and yeast powder used are higher, with no significant increase in the bacterial counts compared to Examples 12 to 16.

Based on the results of the small-scale fermentation tank experiments and cost considerations, a liquid medium containing 0.5 to 1 wt% of glucose, 1 to 2.5 wt% of yeast powder, 0.1 to 0.3 wt% of potassium nitrate, 0.01 to 0.05 wt% of potassium chloride, 0.005 to 0.010 wt% of calcium carbonate, and 0.05 to 0.15 wt% of magnesium chloride is selected for performing a subsequent large-scale fermentation tank experiment.

**(2) Large-Scale Fermentation Tank Experiment**

Production of Fermentation Broth

The seed broths of *Bacillus velezensis* strain (CCTCC, accession number M20211597) having a bacterial count of $1 \times 10^7$ to $1 \times 10^8$ CFU/mL and of $1 \times 10^8$ to $1 \times 10^9$ CFU/mL are added to liquid media and fermented in 10-ton fermentation tanks for 4 to 5 days to obtain fermentation broths. The liquid media composition ratios, fermented temperatures, stirring rates, and inoculation ratios are shown in Table 2 to Table 5. The bacterial count of each fermentation broth is calculated in the same way as in the small-scale fermentation tank experiment, and the results are shown in Table 2 to Table 5.

As shown in Table 2 to Table 5, each of the liquid media used in the Examples and Comparative Examples contains 0.3 wt% of potassium nitrate, 0.05 wt% of potassium chloride, 0.01 wt% of calcium carbonate, and 0.07 wt% of magnesium chloride.

Table 2 shows the concentrations of the fermentation broths produced at different temperatures, stirring rates, and inoculation ratios in Examples 1 to 6 and Comparative Examples 1 to 19. Each of the liquid media contains 0.5 wt% of glucose and 2.5 wt% of yeast powder, and the seed broths have a bacterial count ranging from $10^7$ to $10^8$ cfu/mL. Table 3 shows the concentrations of the fermentation broths produced at different temperatures, stirring rates, and inoculation ratios in Examples 7 to 14 and Comparative Examples 20 to 38. Each of the liquid media contains 1 wt% of glucose and 1 wt% of yeast powder, and the seed broths have a bacterial count ranging from $10^7$ to $10^8$ cfu/mL. Table 4 shows the concentrations of the fermentation broths produced at different temperatures, stirring rates, and inoculation ratios in Examples 15 to 22 and Comparative Examples 39 to 57. Each of the liquid media contains 1 wt% of glucose and 2.5 wt% of yeast powder, and the seed broths have a bacterial count ranging from $10^7$ to $10^8$ cfu/mL. Table 5 shows the concentrations of the fermentation broths produced at different temperatures, stirring rates, and inoculation ratios in Examples 23 to 30. Each of the liquid media contains 1 wt% of glucose and 2.5 wt% of yeast powder, and the seed broths have a bacterial count ranging from $10^8$ to $10^9$ cfu/mL.

Table 2

| | liquid medium composition ratio | | | | | | condition | | | | FBC |
|---|---|---|---|---|---|---|---|---|---|---|---|
| | A (wt%) | B (wt%) | C (wt%) | D (wt%) | E (wt%) | F (wt%) | T (°C) | SR (rpm) | IR (%) | BCSB (cfu/mL) | BC (cfu/mL) |
| Ex. 1 | 0.5 | 2.5 | 0.3 | 0.05 | 0.01 | 0.07 | 30 | 40 | 1 | $10^7$-$10^8$ | $3.50\times10^9$ |
| Ex. 2 | 0.5 | 2.5 | 0.3 | 0.05 | 0.01 | 0.07 | 30 | 40 | 10 | $10^7$-$10^8$ | $2.30\times10^9$ |
| Ex.3 | 0.5 | 2.5 | 0.3 | 0.05 | 0.01 | 0.07 | 30 | 70 | 1 | $10^7$-$10^8$ | $4.50\times10^9$ |
| Ex. 4 | 0.5 | 2.5 | 0.3 | 0.05 | 0.01 | 0.07 | 30 | 70 | 10 | $10^7$-$10^8$ | $2.50\times10^9$ |
| Ex. 5 | 0.5 | 2.5 | 0.3 | 0.05 | 0.01 | 0.07 | 35 | 40 | 1 | $10^7$-$10^8$ | $2.00\times10^9$ |
| Ex. 6 | 0.5 | 2.5 | 0.3 | 0.05 | 0.01 | 0.07 | 35 | 40 | 10 | $10^7$-$10^8$ | $1.10\times10^9$ |
| Com. 1 | 0.5 | 2.5 | 0.3 | 0.05 | 0.01 | 0.07 | 35 | 70 | 0.1 | $10^7$-$10^8$ | $9.00\times10^8$ |
| Com. 2 | 0.5 | 2.5 | 0.3 | 0.05 | 0.01 | 0.07 | 35 | 40 | 0.1 | $10^7$-$10^8$ | $7.00\times10^8$ |
| Com. 3 | 0.5 | 2.5 | 0.3 | 0.05 | 0.01 | 0.07 | 35 | 10 | 0.1 | $10^7$-$10^8$ | $9.00\times10^8$ |
| Com. 4 | 0.5 | 2.5 | 0.3 | 0.05 | 0.01 | 0.07 | 35 | 10 | 1 | $10^7$-$10^8$ | $7.40\times10^8$ |
| Com. 5 | 0.5 | 2.5 | 0.3 | 0.05 | 0.01 | 0.07 | 35 | 10 | 10 | $10^7$-$10^8$ | $8.90\times10^8$ |
| Com. 6 | 0.5 | 2.5 | 0.3 | 0.05 | 0.01 | 0.07 | 30 | 70 | 0.1 | $10^7$-$10^8$ | $9.00\times10^8$ |
| Com. 7 | 0.5 | 2.5 | 0.3 | 0.05 | 0.01 | 0.07 | 30 | 40 | 0.1 | $10^7$-$10^8$ | $9.00\times10^8$ |
| Com. 8 | 0.5 | 2.5 | 0.3 | 0.05 | 0.01 | 0.07 | 30 | 10 | 0.1 | $10^7$-$10^8$ | $6.00\times10^8$ |
| Com. 9 | 0.5 | 2.5 | 0.3 | 0.05 | 0.01 | 0.07 | 30 | 10 | 1 | $10^7$-$10^8$ | $7.40\times10^8$ |
| Com. 10 | 0.5 | 2.5 | 0.3 | 0.05 | 0.01 | 0.07 | 30 | 10 | 10 | $10^7$-$10^8$ | $8.00\times10^8$ |
| Com. 11 | 0.5 | 2.5 | 0.3 | 0.05 | 0.01 | 0.07 | 25 | 70 | 0.1 | $10^7$-$10^8$ | $7.20\times10^8$ |
| Com. 12 | 0.5 | 2.5 | 0.3 | 0.05 | 0.01 | 0.07 | 25 | 70 | 1 | $10^7$-$10^8$ | $7.50\times10^8$ |
| Com. 13 | 0.5 | 2.5 | 0.3 | 0.05 | 0.01 | 0.07 | 25 | 70 | 10 | $10^7$-$10^8$ | $6.20\times10^8$ |
| Com. 14 | 0.5 | 2.5 | 0.3 | 0.05 | 0.01 | 0.07 | 25 | 40 | 0.1 | $10^7$-$10^8$ | $5.50\times10^8$ |
| Com. 15 | 0.5 | 2.5 | 0.3 | 0.05 | 0.01 | 0.07 | 25 | 40 | 1 | $10^7$-$10^8$ | $8.50\times10^8$ |
| Com. 16 | 0.5 | 2.5 | 0.3 | 0.05 | 0.01 | 0.07 | 25 | 40 | 10 | $10^7$-$10^8$ | $6.90\times10^8$ |
| Com. 17 | 0.5 | 2.5 | 0.3 | 0.05 | 0.01 | 0.07 | 25 | 10 | 0.1 | $10^7$-$10^8$ | $9.00\times10^7$ |
| Com. 18 | 0.5 | 2.5 | 0.3 | 0.05 | 0.01 | 0.07 | 25 | 10 | 1 | $10^7$-$10^8$ | $6.80\times10^8$ |
| Com. 19 | 0.5 | 2.5 | 0.3 | 0.05 | 0.01 | 0.07 | 25 | 10 | 10 | $10^7$-$10^8$ | $9.00\times10^7$ |

In Table 2 and the following Tables 3 to 5, the A, B, C, D, E, F, T, SR, IR, BCSB, BC, and FBC represent glucose, yeast powder, potassium nitrate, potassium chloride, calcium carbonate, magnesium chloride, temperature, stirring rate, inoculation ratio, bacterial count of seed broth, bacterial count, and fermentation broth concentration, respectively.

Table 3

| | liquid medium composition ratio | | | | | | condition | | | | FBC |
|---|---|---|---|---|---|---|---|---|---|---|---|
| | A (wt%) | B (wt%) | C (wt%) | D (wt%) | E (wt%) | F (wt%) | T (°C) | SR (rpm) | IR (%) | BCSB (cfu/mL) | BC (cfu/mL) |
| Ex. 7 | 1 | 1 | 0.3 | 0.05 | 0.01 | 0.07 | 30 | 40 | 1 | $10^7$-$10^8$ | $1.10\times10^9$ |
| Ex. 8 | 1 | 1 | 0.3 | 0.05 | 0.01 | 0.07 | 30 | 40 | 10 | $10^7$-$10^8$ | $1.60\times10^9$ |

(continued)

| | liquid medium composition ratio | | | | | | condition | | | | FBC |
|---|---|---|---|---|---|---|---|---|---|---|---|
| | A (wt%) | B (wt%) | C (wt%) | D (wt%) | E (wt%) | F (wt%) | T (°C) | SR (rpm) | IR (%) | BCSB (cfu/mL) | BC (cfu/mL) |
| Ex. 9 | 1 | 1 | 0.3 | 0.05 | 0.01 | 0.07 | 30 | 70 | 1 | $10^7$-$10^8$ | $1.50 \times 10^9$ |
| Ex. 10 | 1 | 1 | 0.3 | 0.05 | 0.01 | 0.07 | 30 | 70 | 10 | $10^7$-$10^8$ | $1.40 \times 10^9$ |
| Ex. 11 | 1 | 1 | 0.3 | 0.05 | 0.01 | 0.07 | 35 | 40 | 1 | $10^7$-$10^8$ | $1.20 \times 10^9$ |
| Ex. 12 | 1 | 1 | 0.3 | 0.05 | 0.01 | 0.07 | 35 | 40 | 10 | $10^7$-$10^8$ | $1.60 \times 10^9$ |
| Ex. 13 | 1 | 1 | 0.3 | 0.05 | 0.01 | 0.07 | 35 | 70 | 1 | $10^7$-$10^8$ | $1.50 \times 10^9$ |
| Ex. 14 | 1 | 1 | 0.3 | 0.05 | 0.01 | 0.07 | 35 | 70 | 10 | $10^7$-$10^8$ | $1.20 \times 10^9$ |
| Com. 20 | 1 | 1 | 0.3 | 0.05 | 0.01 | 0.07 | 35 | 70 | 0.1 | $10^7$-$10^8$ | $1.50 \times 10^8$ |
| Com. 21 | 1 | 1 | 0.3 | 0.05 | 0.01 | 0.07 | 35 | 40 | 0.1 | $10^7$-$10^8$ | $1.20 \times 10^8$ |
| Com. 22 | 1 | 1 | 0.3 | 0.05 | 0.01 | 0.07 | 35 | 10 | 0.1 | $10^7$-$10^8$ | $3.10 \times 10^8$ |
| Com. 23 | 1 | 1 | 0.3 | 0.05 | 0.01 | 0.07 | 35 | 10 | 1 | $10^7$-$10^8$ | $8.80 \times 10^8$ |
| Com. 24 | 1 | 1 | 0.3 | 0.05 | 0.01 | 0.07 | 35 | 10 | 10 | $10^7$-$10^8$ | $7.50 \times 10^8$ |
| Com. 25 | 1 | 1 | 0.3 | 0.05 | 0.01 | 0.07 | 30 | 70 | 0.1 | $10^7$-$10^8$ | $4.50 \times 10^8$ |
| Com. 26 | 1 | 1 | 0.3 | 0.05 | 0.01 | 0.07 | 30 | 40 | 0.1 | $10^7$-$10^8$ | $5.60 \times 10^8$ |
| Com. 27 | 1 | 1 | 0.3 | 0.05 | 0.01 | 0.07 | 30 | 10 | 0.1 | $10^7$-$10^8$ | $2.30 \times 10^8$ |
| Com. 28 | 1 | 1 | 0.3 | 0.05 | 0.01 | 0.07 | 30 | 10 | 1 | $10^7$-$10^8$ | $9.50 \times 10^8$ |
| Com. 29 | 1 | 1 | 0.3 | 0.05 | 0.01 | 0.07 | 30 | 10 | 10 | $10^7$-$10^8$ | $6.00 \times 10^8$ |
| Com. 30 | 1 | 1 | 0.3 | 0.05 | 0.01 | 0.07 | 25 | 70 | 0.1 | $10^7$-$10^8$ | $8.90 \times 10^7$ |
| Com. 31 | 1 | 1 | 0.3 | 0.05 | 0.01 | 0.07 | 25 | 70 | 1 | $10^7$-$10^8$ | $2.20 \times 10^8$ |
| Com. 32 | 1 | 1 | 0.3 | 0.05 | 0.01 | 0.07 | 25 | 70 | 10 | $10^7$-$10^8$ | $3.20 \times 10^8$ |
| Com. 33 | 1 | 1 | 0.3 | 0.05 | 0.01 | 0.07 | 25 | 40 | 0.1 | $10^7$-$10^8$ | $1.20 \times 10^8$ |
| Com. 34 | 1 | 1 | 0.3 | 0.05 | 0.01 | 0.07 | 25 | 40 | 1 | $10^7$-$10^8$ | $4.40 \times 10^8$ |
| Com. 35 | 1 | 1 | 0.3 | 0.05 | 0.01 | 0.07 | 25 | 40 | 10 | $10^7$-$10^8$ | $2.30 \times 10^8$ |
| Com. 36 | 1 | 1 | 0.3 | 0.05 | 0.01 | 0.07 | 25 | 10 | 0.1 | $10^7$-$10^8$ | $5.50 \times 10^7$ |
| Com. 37 | 1 | 1 | 0.3 | 0.05 | 0.01 | 0.07 | 25 | 10 | 1 | $10^7$-$10^8$ | $7.80 \times 10^8$ |
| Com. 38 | 1 | 1 | 0.3 | 0.05 | 0.01 | 0.07 | 25 | 10 | 10 | $10^7$-$10^8$ | $1.10 \times 10^8$ |

Table 4

| | liquid medium composition ratio | | | | | | condition | | | | FBC |
|---|---|---|---|---|---|---|---|---|---|---|---|
| | A (wt%) | B (wt%) | C (wt%) | D (wt%) | E (wt%) | F (wt%) | T (°C) | SR (rpm) | IR (%) | BCSB (cfu/mL) | BC (cfu/mL) |
| Ex. 15 | 1 | 2.5 | 0.3 | 0.05 | 0.01 | 0.07 | 30 | 40 | 1 | $10^7$-$10^8$ | $5.50 \times 10^9$ |
| Ex. 16 | 1 | 2.5 | 0.3 | 0.05 | 0.01 | 0.07 | 30 | 40 | 10 | $10^7$-$10^8$ | $5.80 \times 10^9$ |
| Ex. 17 | 1 | 2.5 | 0.3 | 0.05 | 0.01 | 0.07 | 30 | 70 | 1 | $10^7$-$10^8$ | $5.00 \times 10^9$ |
| Ex. 18 | 1 | 2.5 | 0.3 | 0.05 | 0.01 | 0.07 | 30 | 70 | 10 | $10^7$-$10^8$ | $5.90 \times 10^9$ |
| Ex. 19 | 1 | 2.5 | 0.3 | 0.05 | 0.01 | 0.07 | 35 | 40 | 1 | $10^7$-$10^8$ | $4.90 \times 10^9$ |
| Ex. 20 | 1 | 2.5 | 0.3 | 0.05 | 0.01 | 0.07 | 35 | 40 | 10 | $10^7$-$10^8$ | $5.80 \times 10^9$ |
| Ex. 21 | 1 | 2.5 | 0.3 | 0.05 | 0.01 | 0.07 | 35 | 70 | 1 | $10^7$-$10^8$ | $4.70 \times 10^9$ |
| Ex. 22 | 1 | 2.5 | 0.3 | 0.05 | 0.01 | 0.07 | 35 | 70 | 10 | $10^7$-$10^8$ | $5.75 \times 10^9$ |

(continued)

| | liquid medium composition ratio | | | | | | condition | | | | FBC |
|---|---|---|---|---|---|---|---|---|---|---|---|
| | A (wt%) | B (wt%) | C (wt%) | D (wt%) | E (wt%) | F (wt%) | T (°C) | SR (rpm) | IR (%) | BCSB (cfu/mL) | BC (cfu/mL) |
| Com. 39 | 1 | 2.5 | 0.3 | 0.05 | 0.01 | 0.07 | 35 | 70 | 0.1 | $10^7$-$10^8$ | $9.20 \times 10^8$ |
| Com. 40 | 1 | 2.5 | 0.3 | 0.05 | 0.01 | 0.07 | 35 | 40 | 0.1 | $10^7$-$10^8$ | $9.50 \times 10^8$ |
| Com. 41 | 1 | 2.5 | 0.3 | 0.05 | 0.01 | 0.07 | 35 | 10 | 0.1 | $10^7$-$10^8$ | $9.20 \times 10^8$ |
| Com. 42 | 1 | 2.5 | 0.3 | 0.05 | 0.01 | 0.07 | 35 | 10 | 1 | $10^7$-$10^8$ | $8.90 \times 10^8$ |
| Com. 43 | 1 | 2.5 | 0.3 | 0.05 | 0.01 | 0.07 | 35 | 10 | 10 | $10^7$-$10^8$ | $9.10 \times 10^8$ |
| Com. 44 | 1 | 2.5 | 0.3 | 0.05 | 0.01 | 0.07 | 30 | 70 | 0.1 | $10^7$-$10^8$ | $8.80 \times 10^8$ |
| Com. 45 | 1 | 2.5 | 0.3 | 0.05 | 0.01 | 0.07 | 30 | 40 | 0.1 | $10^7$-$10^8$ | $9.00 \times 10^8$ |
| Com. 46 | 1 | 2.5 | 0.3 | 0.05 | 0.01 | 0.07 | 30 | 10 | 0.1 | $10^7$-$10^8$ | $9.00 \times 10^8$ |
| Com. 47 | 1 | 2.5 | 0.3 | 0.05 | 0.01 | 0.07 | 30 | 10 | 1 | $10^7$-$10^8$ | $8.90 \times 10^8$ |
| Com. 48 | 1 | 2.5 | 0.3 | 0.05 | 0.01 | 0.07 | 30 | 10 | 10 | $10^7$-$10^8$ | $9.40 \times 10^8$ |
| Com. 49 | 1 | 2.5 | 0.3 | 0.05 | 0.01 | 0.07 | 25 | 70 | 0.1 | $10^7$-$10^8$ | $7.80 \times 10^8$ |
| Com. 50 | 1 | 2.5 | 0.3 | 0.05 | 0.01 | 0.07 | 25 | 70 | 1 | $10^7$-$10^8$ | $9.50 \times 10^8$ |
| Com. 51 | 1 | 2.5 | 0.3 | 0.05 | 0.01 | 0.07 | 25 | 70 | 10 | $10^7$-$10^8$ | $9.20 \times 10^8$ |
| Com. 52 | 1 | 2.5 | 0.3 | 0.05 | 0.01 | 0.07 | 25 | 40 | 0.1 | $10^7$-$10^8$ | $8.50 \times 10^8$ |
| Com. 53 | 1 | 2.5 | 0.3 | 0.05 | 0.01 | 0.07 | 25 | 40 | 1 | $10^7$-$10^8$ | $8.90 \times 10^8$ |
| Com. 54 | 1 | 2.5 | 0.3 | 0.05 | 0.01 | 0.07 | 25 | 40 | 10 | $10^7$-$10^8$ | $8.90 \times 10^8$ |
| Com. 55 | 1 | 2.5 | 0.3 | 0.05 | 0.01 | 0.07 | 25 | 10 | 0.1 | $10^7$-$10^8$ | $1.30 \times 10^8$ |
| Com. 56 | 1 | 2.5 | 0.3 | 0.05 | 0.01 | 0.07 | 25 | 10 | 1 | $10^7$-$10^8$ | $8.80 \times 10^8$ |
| Com. 57 | 1 | 2.5 | 0.3 | 0.05 | 0.01 | 0.07 | 25 | 10 | 10 | $10^7$-$10^8$ | $6.00 \times 10^8$ |

Table 5

| | liquid medium composition ratio | | | | | | condition | | | | FBC |
|---|---|---|---|---|---|---|---|---|---|---|---|
| | A (wt%) | B (wt%) | C (wt%) | D (wt%) | E (wt%) | F (wt%) | T (°C) | SR (rpm) | IR (%) | BCSB (cfu/mL) | BC (cfu/mL) |
| Ex. 23 | 1 | 2.5 | 0.3 | 0.05 | 0.01 | 0.07 | 30 | 40 | 1 | $10^8$-$10^9$ | $2.70 \times 10^{10}$ |
| Ex. 24 | 1 | 2.5 | 0.3 | 0.05 | 0.01 | 0.07 | 30 | 40 | 10 | $10^8$-$10^9$ | $2.85 \times 10^{10}$ |
| Ex. 25 | 1 | 2.5 | 0.3 | 0.05 | 0.01 | 0.07 | 30 | 70 | 1 | $10^8$-$10^9$ | $2.60 \times 10^{10}$ |
| Ex. 26 | 1 | 2.5 | 0.3 | 0.05 | 0.01 | 0.07 | 30 | 70 | 10 | $10^8$-$10^9$ | $2.97 \times 10^{10}$ |
| Ex. 27 | 1 | 2.5 | 0.3 | 0.05 | 0.01 | 0.07 | 35 | 40 | 1 | $10^8$-$10^9$ | $2.50 \times 10^{10}$ |
| Ex. 28 | 1 | 2.5 | 0.3 | 0.05 | 0.01 | 0.07 | 35 | 40 | 10 | $10^8$-$10^9$ | $2.80 \times 10^{10}$ |
| Ex. 29 | 1 | 2.5 | 0.3 | 0.05 | 0.01 | 0.07 | 35 | 70 | 1 | $10^8$-$10^9$ | $2.60 \times 10^{10}$ |
| Ex. 30 | 1 | 2.5 | 0.3 | 0.05 | 0.01 | 0.07 | 35 | 70 | 10 | $10^8$-$10^9$ | $2.81 \times 10^{10}$ |

It can be seen obviously from the results shown in Table 2 that the fermentation broths having bacterial counts of more than $1 \times 10^9$ CFU/mL can be produced in Examples 1 to 6, each of which uses the liquid medium of the present invention (comprising 0.5 wt% of glucose, 2.5 wt% of yeast powder, 0.3 wt% of potassium nitrate, 0.05 wt% of potassium chloride, 0.01 wt% of calcium carbonate, and 0.07 wt% of magnesium chloride) and is carried out at a temperature of 30 to 35°C and at a stirring rate of 40 to 70 rpm along with the inoculation ratio ranging from 1 to 10%. In contrast, all the fermentation broths produced in Comparative Examples 1 to 19, where the temperatures, stirring rates, and inoculation ratios are outside the ranges mentioned above, have bacterial counts below $10^9$ CFU/mL. Similarly, as shown in Table 3 and Table 4, the fermentation broths produced in Examples 7 to 14 and Examples 15 to 22 have bacterial counts of more than $1 \times 10^9$ CFU/mL. Each of the foregoing Examples uses the liquid medium of the present invention, which comprises 1 wt% of glucose, 1 wt% of yeast powder, 0.3 wt% of potassium nitrate, 0.05 wt% of potassium chloride, 0.01 wt% of calcium carbonate, and 0.07 wt% of magnesium chloride, or comprises 1 wt% of glucose, 2.5 wt% of yeast powder, 0.3 wt% of potassium nitrate, 0.05 wt% of potassium chloride, 0.01 wt% of calcium carbonate, and 0.07 wt% of magnesium chloride, and is carried out at a temperature of 30 to 35°C and at a stirring rate of 40 to 70 rpm along with the inoculation ratio ranging from 1 to 10%. In contrast, all the fermentation broths produced in Comparative Examples 20 to 38 and Comparative Examples 39 to 57, where the temperatures, stirring rates, and inoculation ratios are outside the ranges mentioned above, have a maximum bacterial count of only $10^8$ CFU/mL. Furthermore, as shown in Table 5, in addition to using the liquid medium of the present invention (comprising 1 wt% of glucose, 2.5 wt% of yeast powder, 0.3 wt% of potassium nitrate, 0.05 wt% of potassium chloride, 0.01 wt% of calcium carbonate, and 0.07 wt% of magnesium chloride), setting the temperature at 30 to 35°C, the stirring rate at 40 to 70 rpm, the inoculation ratio at 1 to 10%, and using the seed broth having a bacterial count of $10^8$ to $10^9$ CFU/mL, all the fermentation broths produced in Examples 23 to 30 achieve bacterial counts as high as $10^{10}$ CFU/mL.

### (3) **Storage Stability of Fermentation Broth of** *Bacillus Velezensis* **Strain**

The fermentation broth of *Bacillus velezensis* strain produced in a 10-ton fermentation tank using the liquid medium and culture condition of the invention, has an initial bacterial count of $5 \times 10^9$ CFU/mL. After storage at room temperature (approximately 25 to 30°C) for two years, the bacterial count in the fermentation broth remains at $5 \times 10^9$ CFU/mL without significant change, demonstrating excellent storage stability.

In summary, the production method for *Bacillus velezensis* strain fermentation broth provided in the present invention is not only suitable for ton-scale fermentation equipment, but also enables the production of fermentation broth having a bacterial count of more than $1 \times 10^9$ CFU/mL, and even reaching $10^{10}$ CFU/mL in a cost-effective manner. Additionally, the produced fermentation broth exhibits excellent storage stability, which is beneficial for subsequent applications of microbial pesticides and also meets the industrial mass production requirements.

### Claims

1. A production method of a fermentation broth with *Bacillus velezensis* strain, **characterized in that** the production method comporises the steps of:

    inoculating a seed broth of *Bacillus velezensis* strain having a bacterial count of $1 \times 10^7$ to $1 \times 10^9$ CFU/mL at an inoculation ratio of 1% to 10% into a liquid medium, and culturing in a ton-scale fermentation tank at a temperature of 30°C to 35°C and at a stirring rate of 40 to 70 rpm for 4 to 5 days, to obtain a fermentation broth having a bacterial count of more than $1 \times 10^9$ CFU/mL;
    wherein said *Bacillus velezensis* strain is deposited at the China Center For Type Culture Collection (CCTCC) under accession number M20211597; and
    wherein said liquid medium comprises 0.5 to 1 wt% of carbon source, 1 to 2.5 wt% of nitrogen source, 0.1 to 0.3 wt% of nitrate compound, 0.01 to 0.05 wt% of chlorine source, 0.005 to 0.010 wt% of calcium source, 0.05 to 0.15 wt% of magnesium source, and a balance of deionized water.

2. The production method of claim 1, **characterized in that** the obtained fermentation broth has a bacterial count of less than $3 \times 10^{10}$ CFU/mL.

3. The production method of claim 1, **characterized in that** the carbon source contained in said liquid medium is at least one selected from the group consisting of glucose, molasses, granulated sugar, lactose, and corn starch; the nitrogen source contained in said liquid medium is at least one selected from the group consisting of yeast powder, full-fat soybean powder, defatted soybean powder, soybean protein, and peptone.

4. The production method of claim 1, **characterized in that** the nitrate compound contained in said liquid medium is ammonium nitrate, potassium nitrate, or a mixture thereof; the chlorine source contained in said liquid medium is at least one selected from the group consisting of sodium chloride, ferric chloride, and potassium chloride; the calcium source contained in said liquid medium is calcium carbonate, monocalcium phosphate, or a mixture thereof; the magnesium source contained in said liquid medium is magnesium chloride, magnesium sulfate, or a mixture thereof.

5. The production method of claim 1, **characterized in that** the obtained fermentation broth has a bacterial count ranging

from $1\times10^{10}$ CFU/mL to $3\times10^{10}$ CFU/mL, provided that said seed broth has a bacterial count of $1\times10^8$ to $1\times10^9$ CFU/mL, and said liquid medium comprises 1 wt% of glucose, 2.5 wt% of yeast powder, 0.05 wt% of potassium chloride, 0.3 wt% of potassium nitrate, 0.010 wt% of calcium carbonate, 0.07 wt% of magnesium chloride, and a balance of deionized water.

6. A *Bacillus velezensis* strain fermentation broth, **characterized in that** the *Bacillus velezensis* strain fermentation broth is obtained by the production method of any one of claims 1 to 5.

7. A liquid medium for use in the production method of any one of claims 1 to 5, **characterized in that** said liquid medium comprises:

0.5 to 1 wt% of carbon source, 1 to 2.5 wt% of nitrogen source, 0.1 to 0.3 wt% of nitrate compound, 0.01 to 0.05 wt% of chlorine source, 0.005 to 0.010 wt% of calcium source, 0.05 to 0.15 wt% of magnesium source, and a balance of deionized water.

8. The liquid medium of claim 7, **characterized in that** the carbon source is at least one selected from the group consisting of glucose, molasses, granulated sugar, lactose, and corn starch; the nitrogen source is at least one selected from the group consisting of yeast powder, full-fat soybean powder, defatted soybean powder, soybean protein, and peptone.

9. The liquid medium of claim 7, **characterized in that** the nitrate compound is ammonium nitrate, potassium nitrate, or a mixture thereof; the chlorine source is at least one selected from the group consisting of sodium chloride, ferric chloride, and potassium chloride; the calcium source is calcium carbonate, monocalcium phosphate, or a mixture thereof; the magnesium source is magnesium chloride, magnesium sulfate, or a mixture thereof.

10. The liquid medium of claim 7, **characterized in that** the carbon source is 0.5 to 1 wt% of glucose, the nitrogen source is 1 to 2.5 wt% of yeast powder, the nitrate compound is 0.3 wt% of potassium nitrate, the chlorine source is 0.05 wt% of potassium chloride, the calcium source is 0.01 wt% of calcium carbonate, and the magnesium source is 0.07 wt% of magnesium chloride.

**EUROPEAN SEARCH REPORT**

Application Number

EP 24 21 4217

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CN 116 064 318 A (YELLOW RIVER DELTA JINGBO RES INSTITUTE OF CHEMICAL INDUSTRY CO LTD) 5 May 2023 (2023-05-05) * See translation * | 1-10 | INV. C12N1/20 |
| A | CN 115 161 239 A (SICHUAN LOMON BIO TECH CO LTD) 11 October 2022 (2022-10-11) * See translation * | 1-10 | |

**TECHNICAL FIELDS SEARCHED (IPC)**

C12N
C12R

The present search report has been drawn up for all claims

| Place of search | Date of completion of the search | Examiner |
|---|---|---|
| Munich | 9 April 2025 | Roscoe, Richard |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

   ........................................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 24 21 4217

This annex lists the patent family members relating to the patent documents cited in the above-mentioned European search report.
The members are as contained in the European Patent Office EDP file on
The European Patent Office is in no way liable for these particulars which are merely given for the purpose of information.

09-04-2025

| Patent document cited in search report | Publication date | Patent family member(s) | Publication date |
|---|---|---|---|
| CN 116064318 A | 05-05-2023 | NONE | |
| CN 115161239 A | 11-10-2022 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- TW I740263